(19) Europäisches Patentamt

European Patent Office

Office européen des brevets

(11) Publication number: **0 285 367 B1**

# EUROPEAN PATENT SPECIFICATION

(45) Date of publication of patent specification: 26.05.93  (51) Int. Cl.5: **A61K 47/00, A61K 9/06**

(21) Application number: **88302772.4**

(22) Date of filing: **29.03.88**

The file contains technical information submitted after the application was filed and not included in this specification

(54) **Pharmaceutical composition.**

(30) Priority: **01.04.87 US 33325**

(43) Date of publication of application:
**05.10.88 Bulletin 88/40**

(45) Publication of the grant of the patent:
**26.05.93 Bulletin 93/21**

(84) Designated Contracting States:
**AT BE CH DE ES FR GB GR IT LI LU NL SE**

(56) References cited:
**EP-A- 0 128 831**
**FR-A- 2 321 275**
**US-A- 4 476 116**

**CHEMICAL ABSTRACTS, vol. 96, no. 5, 1 February 1982, page 20, column 1, abstract no. 28229n, Columbus, Ohio, USA; H. YAGINUMA et al.: "Rectal delivery of antiin-flammatory drugs. I. The influence of antiin-flamatory drugs on rectal absorption of beta-lactam antibiotics"**

**PATENT ABSTRACTS OF JAPAN, vol. 8, no. 114 (C-225)(1551), 26 May 1986; & JP-A-5927978( TERUMO K.K.) 14.02.1984**

The Merck Index (1989) p. 562

(73) Proprietor: **Temple University of the Com-monwealth System of Higher Education**
**1801 North Broad Street**
**Philadelphia Pennsylvania 19122(US)**

(72) Inventor: **Kowarski, Hanna R.**
**2405 Sugracone Road**
**Pikesville Maryland 21209(US)**

(74) Representative: **Ackroyd, Robert et al**
**W.P. THOMPSON & CO. Eastcheap House**
**Central Approach**
**Letchworth, Hertfordshire SG6 3DS (GB)**

Rank Xerox (UK) Business Services
(3.10/3.6/3.3.1)

## Description

The invention relates to a novel compostion and method for the intranasal administration of pharmaceutical substances.

Systemically active drugs have been administered by a wide variety of routes, such as orally, rectally, vaginally, subcutaneously, intramuscularly, intraveneously, etc.

Some pharmaceuticals, peptide drugs in particular, are not suitable for oral administration. For these drugs, parenteral administration is the only alternative.

The traditional mode of administration of insulin is by subcutaneous injection. Control of diabetes mellitus often requires multiple injections each day, which are painful and distressing to many patients.

Discomfort and destruction of lifestyle often deter diabetics from accepting intensive insulin treatment. For these reasons, attention has been focused on alternative routes of administration of insulin and other chronically-needed medicaments.

Attempts have been initiated to administer insulin enterally, with and without liposome encapsulation, rectally, vaginally, and through the respiratory epithelium. Because of the limited absorption of insulin, these attempts have largely failed.

A great deal of interest has been focused on the intranasal mode of administration of drugs. Vasopressin, luteinizing hormone releasing factor ("LHRF"), adrenocorticotrophic hormone ("ACTH"), and in particular insulin, have been administered intranasally. While intranasal administration offers advantages over other routes, many drugs exhibit only limited absorption through the nasal mucosa. For example, insulin, when administered intranasally, neither increases serum insulin levels nor lowers blood glucose concentration. To be absorbed from the nasal mucosa to reach the blood circulation, the pharmaceutical molecules must be transported across nasal mucous membranes by means of an absorption enhancer.

Many agents have been suggested as intranasal absorption enhancers. Patent 4,476,116 discloses pharmaceutical compositions for intranasal delivery including chelating agents which enhance absorption across the nasal mucous membranes. Patent 4,153,689 discloses insulin preparations for intranasal administration containing one or more non-ionic surface-active agents as an absorption enhancer. Bile salts such as sodium deoxycholate have also been used to increase intranasal insulin absorption. Moses et al, Diabetes 32: 1040-1047 (1983); Gordon et al, Proc. Natl. Acad. Sci. USA 82:7419 (1985). However, bile salts are undesirable since they cause nasal irritation and damage to the nasal mucosa. While much attention has been devoted to intranasal insulin formulations containing non-ionic surfactant enhancers such as laureth-9 (polyoxethylene-9-laurylether), such surfactants cause nasal stinging, congestion and rhinorrhea. Salzman et al, N. Eng. J. Med. 312: 1078-1084 (1985).

US-A-4476116 (Anik) discloses a nasal spray composition comprising a polypeptide as the active ingredient, an absorption-enhancing chelating agent and an excipient, such as an anti-oxidant.

Patent Abstracts of Japan, Vol. 8, No. 114 (Terumo) discloses a transdermal device comprising an impermeable backer film on to which is formed an adhesive layer containing indomethacin and a penetration assistant, exemplified by glycyrrhetinic acid.

What is needed is an effective enhancing agent for the transport of pharmaceutically active substances across the nasal membrane which is free of the irritation and other undesirable side effects experienced with presently-used intransasl delivery formulations.

The invention provides a composition for the intranasal administration of pharmaceutically active substances. The composition comprises an effective amount of a pharmaceutically active substance, a basic salt of an amino acid, and glycyrrhetinic acid.

Figure 1 is a plot of the blood glucose concentration of an animal subject as a function of time after nasal administration of 0.1 ml of an insulin preparation according to Example 1.

Figure 2 is a plot of the blood glucose concentration of an animal subject as a function of time after nasal administration of 0.1 ml of an insulin preparation according to Example 2.

Figure 3 is a plot of the blood glucose concentration of an animal subject as a function of time after nasal administration of 0.1 ml of an insulin preparation according to Example 3a.

Figure 4 is a plot of the blood glucose concentration of an animal subject as a function of time after nasal administration of 0.2 ml of an insulin preparation according to Example 3a.

Figure 5 is a plot of the blood glucose concentration of an animal subject as a function of time after nasal administration of 0.2 ml of an insulin preparation according to Example 3b.

Figure 6 is a plot of the blood glucose concentration of an animal subject as a function of time after nasal administration of 0.2 ml of an insulin preparation according to Example 3c.

Figure 7 is a plot of the blood glucose concentration of an animal subject as a function of time after nasal administration of 0.2 ml of an insulin preparation according to Example 8.

Figure 8 is a plot of the blood glucose concentration of an animal subject as a function of time after nasal administration of 0.2 ml of an insulin preparation according to Example 10.

Figure 9 is a plot of the blood glucose concentration of an animal subject as a function of time after nasal administration of 0.1 ml of an insulin preparation according to Example 11.

Figure 10 is a plot of the blood glucose concentration of an animal subject as a function of time after nasal administration of 0.2 ml of an insulin preparation according to Example 11.

Figure 11 is a plot of the blood glucose concentration of an animal subject as a function of time after nasal administration of 0.1 ml of an insulin preparation according to Example 12.

Figure 12 is a plot of the blood glucose concentration of an animal subject as a function of time after nasal administration of 0.2 ml of an insulin preparation according to Example 13.

Figure 13 is a plot of the free insulin plasma concentration of a human subject as a function of time just prior to and after nasal administration of 50 units of an insulin composition according to Example 6 utilizing porcine insulin.

Glycyrrhetinic acid, the aglycone of glycyrrhizin, may exist in a number of isomeric forms, two of which are known as 18 alpha − and 18 beta − glycyrrhetinic acid. I have found surprisingly that when glycyrrhetinic acid is combined with a basic salt of an amino acid in aqueous solution, the resulting composition significantly enhances absorption of pharmaceutical agents across the nasal membrane without the deleterious side effects of known nasal absorption enhancing agents.

Drugs which may be prepared for nasal administration in this manner include insulin, growth hormone, growth hormone releasing factor, glucagon and somatostatin; and interferon; steroids such as prednisone, prednisolone, hydrocortisone, triamcinolone, dexamethasone and betamethasone; antiinflamatory agents such as aspirin, aminopyrine, acetaminophen, ibufenac, ibuprofen, indomethacin, colehicine, sulpyrine, mefenamic acid, phenacetin, phenylbutazone, flufenamic acid and probenecid; antihistamine agents such as diphenhydramine hydrochloride and dexchlorpheniramine maleate; antibiotics such as penicillin or its derivatives, cephalosporin or its derivatives; erythromycin, tetracycline, furadiomycin, leucomycin; chemotherapeutic agents such as sulfathiazole and nitrofurazone; cardiac agents such as digitalis and digoxin; blood vein dialating agents such as nitroglycerin and papaverine hydrochloride; cough curing agents such as codeine; azulen; phenovalin; pepsin; vitamin U; enzymes such as lysozyme hydrochloride; other systemic agents such as anti − hypertensives and diuretics; tranquilizers; sex hormones; and ulcer medications.

Other such pharmaceutical agents are known to those skilled in the art. The intranasal delivery composition is particularly well suited for the following pharmaceuticals presently marketed or under investigation for nasal delivery: vasopressin, oxytocin, luteinizing hormone releasing factor (LHRF), cal − citonin, auriculin, flu vaccine and other vaccines, thyrotrophin releasing hormone (TRH), progesterone, propanolol, metoclopramide, narcotic analgesics, vitamin $B_{12}$ and antihistamines.

The intranasal delivery compostion is also particularly well − suited for the administration of insulin. Insulin may be of the animal type, such as porcine or bovine insulin. Human insulin, such as prepared by recombinant DNA techniques, may also be used. A method for treating diabetes mellitus therefore comprises administering through the nasal mucous membrane of a patient suffering from diabetes an effective amount of an aqueous insulin solution according to the present invention which contains an amount of insulin effective in inducing regulation of blood glucose level.

Any isomer of glycyrrhetinic acid may be used in the compositions of the present invention, although the 18 alpha − and 18 beta − isomers are preferred. 18 beta − glycyrrhetinic acid is particularly preferred. The compositions of the present invention containing glycyrrhetinic acid have no taste or after − effects, unlike prior art intranasal compositons relying on surfactant or bile salt enhancers.

The composition contains one or more basic salts of an amino acid. Amino acids may be conveniently converted to their basic salts by treatment with an appropriate base, such as potassium hydroxide or sodium hydroxide. Suitable amino acid basic salts include, for example, the sodium or potassium salts of glycine, aspartatic acid, and glutamic acid. Either the levo, dextro or racemic forms of the amino acids may be employed. Thus, useful basic salts of amino acids for use in the present compostions include, for example, sodium glycinate, monosodium aspartate, monosodium L − glutamate, monopotassium L − aspar − tate, monopotassium D, L − aspartate, and other basic salts of amino acids.

The pharmaceutical substance, glycyrrhetinic acid in an amount effective to enhance absorption of the pharmaceutical substances across nasal membranes, and the basic amino acid salt may be dissolved in an aqueous dilluent. Water may be used. Alternatively, the dilluent may comprise an aqueous buffer such as phosphate buffer.

A phosphate buffer, pH 7.6, useful as a dilluent may be prepared by combining 2.5 ml of 0.02M $KH_2PO_4$ and 2.12 ml of 0.02 M NaOH, and adding $H_2O$ up to a final volume of 10.0 ml.

3

Generally, a concentration of glycyrrhetinic acid of about 0.25 to about 1.0% (w/v) in the composition will be sufficient to obtain an acceptable absorption enhancement across the nasal membrane. A higher or lower glycyrrhetinic acid concentration may be required, depending on the nature and dose of the pharmaceutical substance being administered. The concentration of amino acid basic salt is preferably that which will establish, on a molar basis, a concentration of basic amino acid salt equal to the concentration of glycyrrhetinic acid in the composition.

The composition may further optionally include one or more polyhydric alcohols to increase the soluability of glycyrrhetinic acid. Such polyhydric alcohols include, for example, propylene glycol, glycerin, polyethylene glycol and sorbitol. A hydroxide, e.g. NaOH, may also be added when needed to increase the alkalinity of the formulation to promote dissolving of glycyrrhetinic acid.

Finally, the composition may optionally include one or more preservative agents such as, for example, gentamycin, bacitracin (0.005%), or cresol.

The preparations of the invention may be produced by mixing the ingredients in any order by conventional means, taking care that the glycyrrhetinic acid becomes dissolved in the diluent.

The compositions may be administered to the nasal cavity in the form of a spray by using an atomiser, nubuliser, sprayer, dropper or other device which insures contact of the solution with the nasal mucous membrane.

The practice of the present invention is illustrated by the following non−limiting examples. For preparations containing human insulin, a commercially available human insulin solution containing 100 units/ml and 0.2% (w/v) phenol was utilized as a source of insulin. Unless stated otherwise, each preparation contains 1% (w/v) glycyrrhetinic acid.

Example 1

100.5 mg 18 alpha−glycyrrhetinic acid are mixed in a test tube with 1 ml of a solution of 501 mg sodium glycinate in 10 ml of 0.02 M phosphate buffer (prepared by combining 2.5 ml 0.02 M $KH_2PO_4$ and 2.12 ml 0.02 M NaOH, and adding water up to a final volume of 10.0 ml). The mixture is stirred with a glass rod and heated to 90−100˚C to dissolve the glycyrrhetinic acid by immersing the test tube in a water bath of boiling water for about 5 min. Stirring may continue until the mixture becomes homogenous. Following removal from the hot water bath, 1.0 ml of propylene glycol is added, followed by 5 drops from a solution of 502 mg glycine HCl in 10 ml of 0.02 M phosphate buffer. 0.02 M phosphate buffer is then added to raise the solution to a volume of 5 ml. One ml of this nasal absorption−enhancing solution is mixed with 1 ml of a 100 unit/ml commercially−available human insulin solution (Squib−Novo). The resulting preparation contains 50 units of insulin per ml.

Example 2

To 106.5 mg 18 alpha−glycyrrhetinic acid are added 1 ml of a solution of 502 mg sodium glycinate in 10 ml of 0.02 M phosphate buffer followed by mixing and heating as in Example 1. 1.5 ml of propylene glycol are then added, followed by 0.02 M phosphate buffer to 5 ml. 2.5 ml of this solution are added to 2.5 ml of a 100 unit/ml solution of human insulin. The insulin concentration in this final preparation is 50 units/ml.

Example 3a

To 100.3 mg 18 beta−glycyrrhetinic acid are added 1 ml of a solution containing 100.5 mg sodium glycinate per ml of distilled water, followed by mixing and heating as in Example 1. 1 ml glycerin is then added, followed by water up to 5 ml. 2 ml of this preparation are added to 2 ml of a human insulin solution of 100 units per ml to yield a final insulin preparation of 50 units per ml insulin.

Example 3b

The preparation of Example 3a is repeated except the amount of glycyrrhetinic acid is reduced to 0.5% (w/v).

### Example 3c

The preparation of Example 3a is repeated except the concentration of glycyrrhetinic acid is reduced to 0.25% (w/v).

### Example 4

To 200.5 mg 18 beta−glycyrrhetinic acid are added 1.5 ml of a solution of 1005 mg sodium glycinate in 10 ml water, followed by mixing and heating as in Example 1. 1 ml of glycerin is then added. 2 ml of this preparation is combined with 2 ml of a human insulin solution containing 100 units per ml of insulin, which yields a final preparation containing 50 units of insulin per ml.

### Example 5

50 mg sodium glycinate are added to 0.5 ml of 0.02 M phosphate buffer and 50 mg 18 beta−glycyrrhetinic acid and mixed until liquid. 0.5 ml of glycerin is then added. 50 mg of insulin crystals (24 units per mg) are dissolved in 1 ml of 0.1 N HCl, which is then added to the above mixture. Addition of phosphate buffer up to 5 ml provides a final preparation containing 240 units of insulin per ml.

### Example 6

0.5 ml of glycerin and 2 ml of water are added to 2.5 ml of the final preparation from Example 4. 5 ml of the resulting solution is combined with 5 ml of a human insulin solution of 500 units per ml. The final preparation contains 250 units of insulin per ml and 0.5% (w/v) of 18 beta−glycyrrhetinic acid.

### Example 7

To 90.5 mg of 18 beta−glycyrrhetinic acid are added 1 ml of a solution consisting of 504 mg L−aspartic acid monosodium salt per ml of water. Following mixing and heating as in Example 1, 0.2 ml of 1 N NaOH, 1 ml of glycerin, and water up to 5 ml are added. The resulting solution is added to 5 ml of an aqueous human insulin solution containing 100 units of insulin per ml to yield a final preparation containing 50 units of insulin per ml.

### Example 8

2 ml of a solution of 501.5 mg L−glutamic acid monosodium salt in 10 ml water are added to 100 mg 18 beta−glycyrrhetinic acid, and mixed and heated as in Example 1. 0.4 ml of 1N NaOH, 1 ml of glycerin and water up to 5 ml are then added. 5 ml of a human 100 unit/ml insulin solution are added to yield a final insulin preparation of 50 units per ml.

### Example 9

1 ml of a solution of 510 mg L−aspartic acid monopotassium salt in 10 ml water is added to 100.5 mg 18 betaglycyrrhetinic acid, followed by mixing and heating as in Example 1. 0.2 ml 1N NaOH solution, 1 ml of glycerin and water up to 5 ml are then added. This solution is combined with 5 ml of a human insulin solution containing 100 units insulin per ml. The final preparation contains 50 units of insulin per ml.

### Example 10

To 100.5 mg 18 beta−glycyrrhetinic acid are added 1 ml of a solution containing 510 mg D,L−aspartic acid monopotassium salt in 10 ml water, followed by mixing and heating as in Example 1. 0.2 ml 1N NaOH, 1 ml of glycerin and water up to 5 ml are then added. This solution is mixed with 5 ml of a human insulin solution containing 100 units per ml insulin to provide a final preparation containing 50 units of insulin per ml.

Comparative Example 11

1 ml of a 100 unit/ml human insulin solution was mixed with 1 ml of distilled water. This diluted solution containing 50 units/ml insulin was used as a control.

Comparative Example 12

1 ml of a human insulin solution containing 100 units/ml of insulin were mixed with 1 ml of phosphate buffer, pH 7.6. The diluted solution of 50 units/ml insulin was used as a control.

Comparative Example 13

A sham preparation was prepared according to Example 3a, but omitting the insulin.

The hypoglycemic effect of the above preparations was confirmed in animal glucose monitoring studies utilizing continuous glucose monitoring with the device described in J. Clin. Endocrinol. Metab. 53: 1145 (1984). The device consists of a system for nonthrombogenic blood withdrawal coupled to a system for blood glucose measurement. The blood withdrawal system includes a disposable sterile intravenous needle and catheter connected to a peristaltic pump. The inside wall of the catheter is coated with tridodecyclmethylammonium chloride complexed with heparin to provide a nonthrombogenic surface. Blood is continuously withdrawn at a rate of 12 ml/hr from the experimental animal via the nonthrombogenic catheter. The blood is diluted in a Plexiglass mixing chamber with six volumes of phosphate buffer solution (0.015 M; pH 7.4) containing 10 IU/ml heparin and then moved into the device's sensory chamber. The sensory chamber contains a glucose-sensing system comprising a glucose sensing probe, a digital display and a digital graphic recorder.

Animal Study

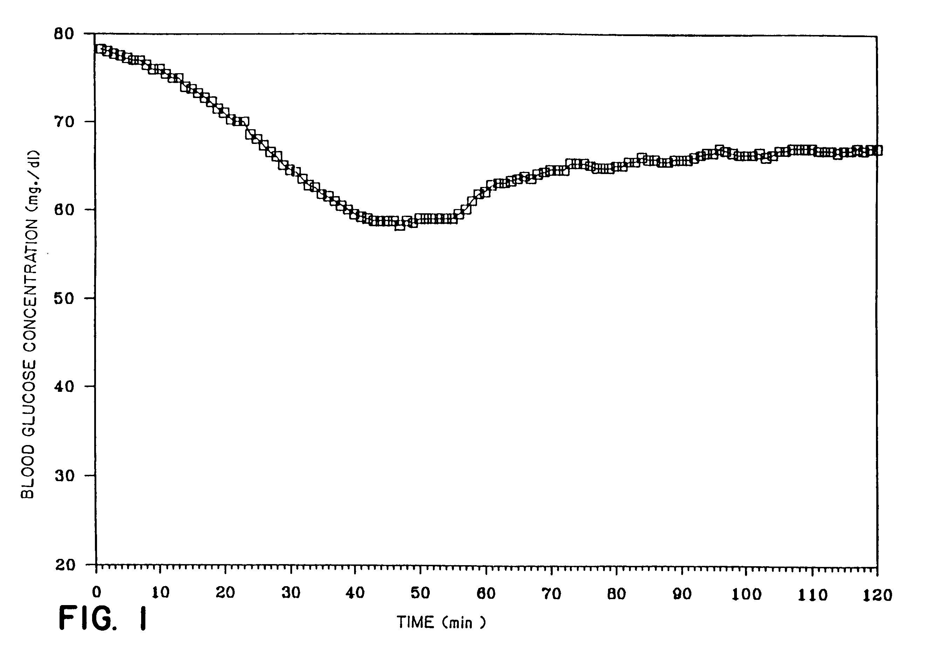

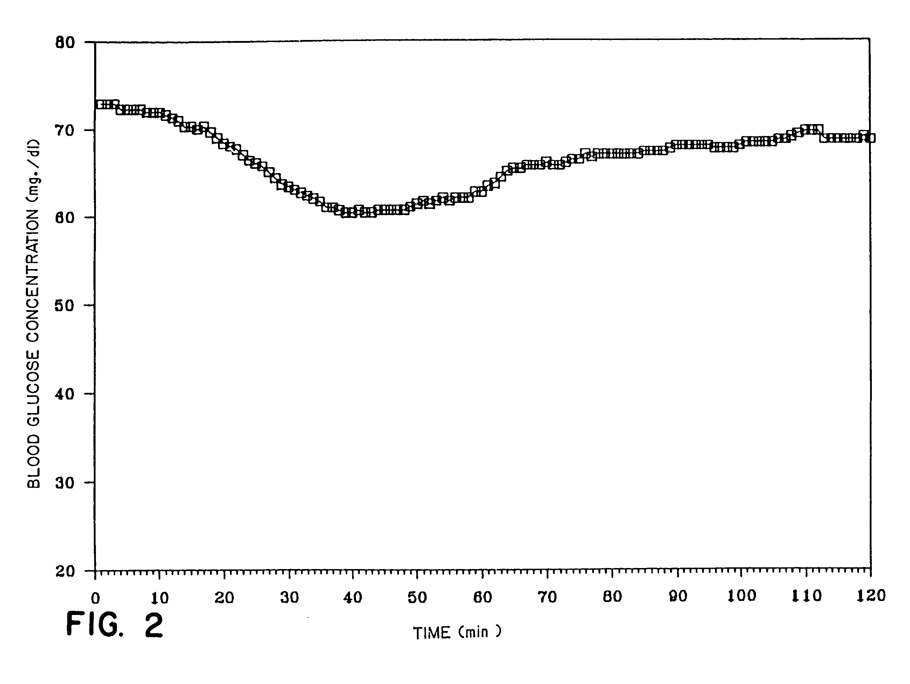

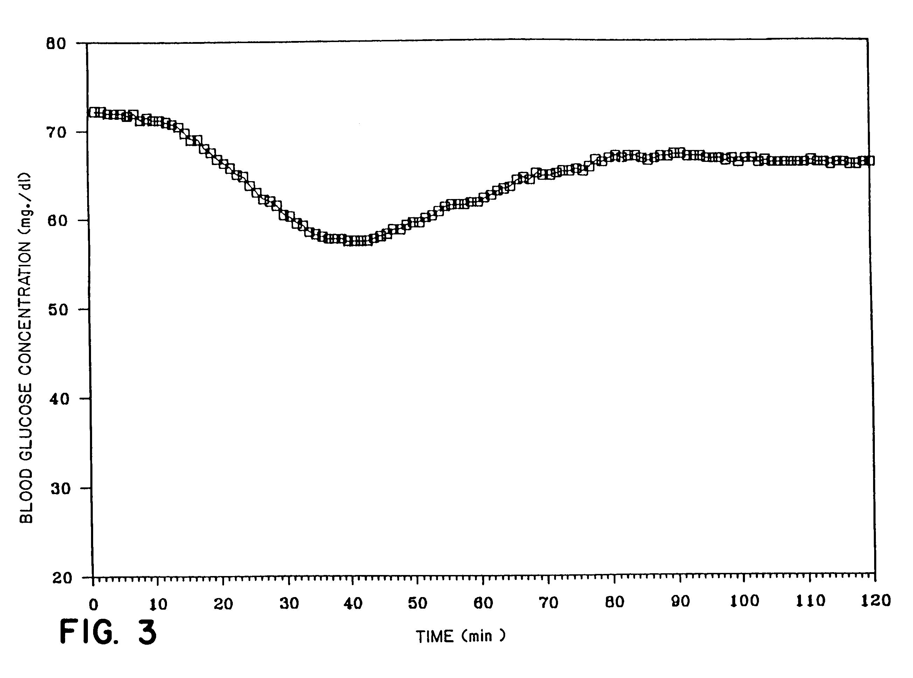

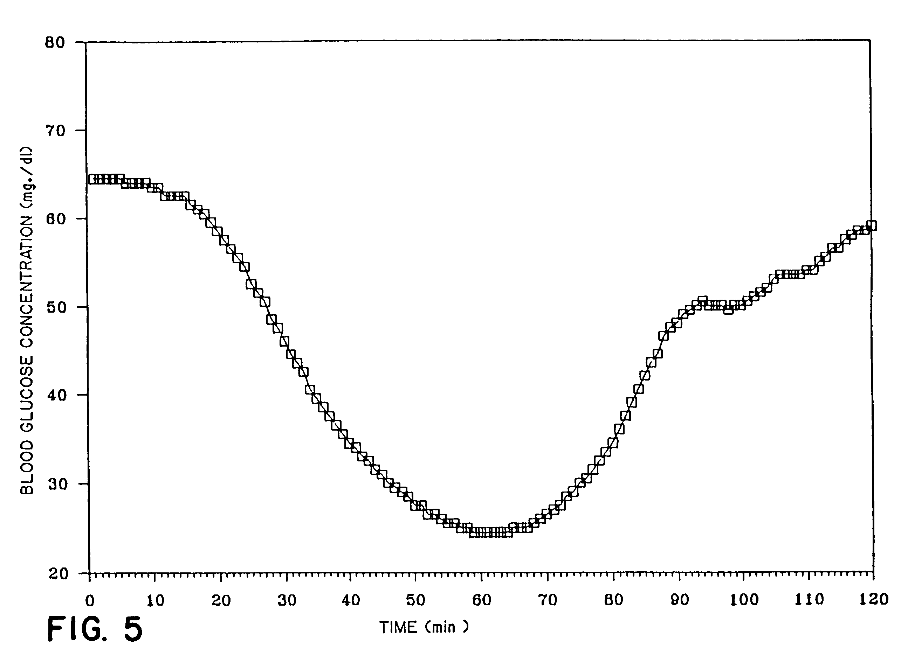

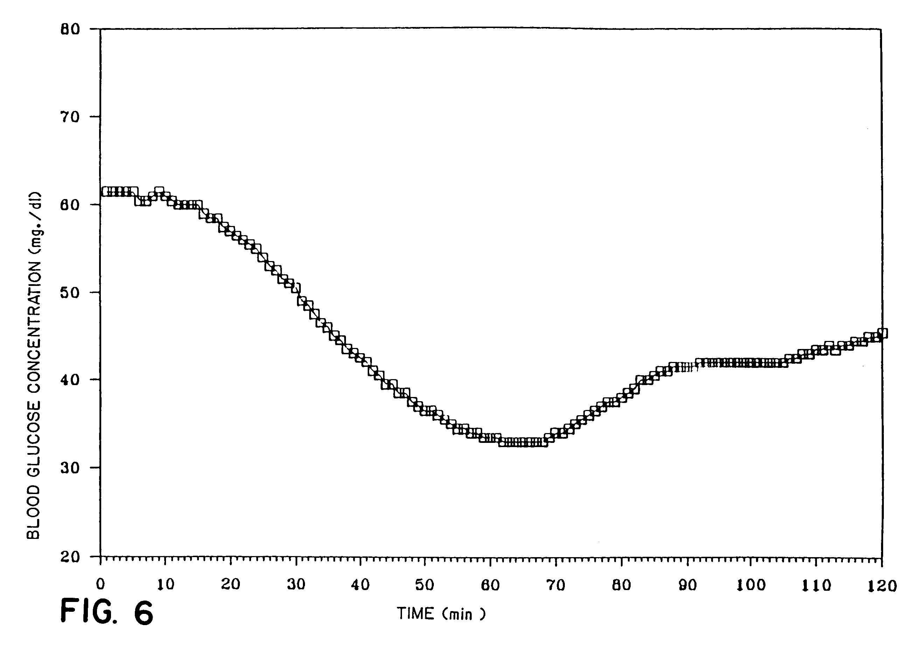

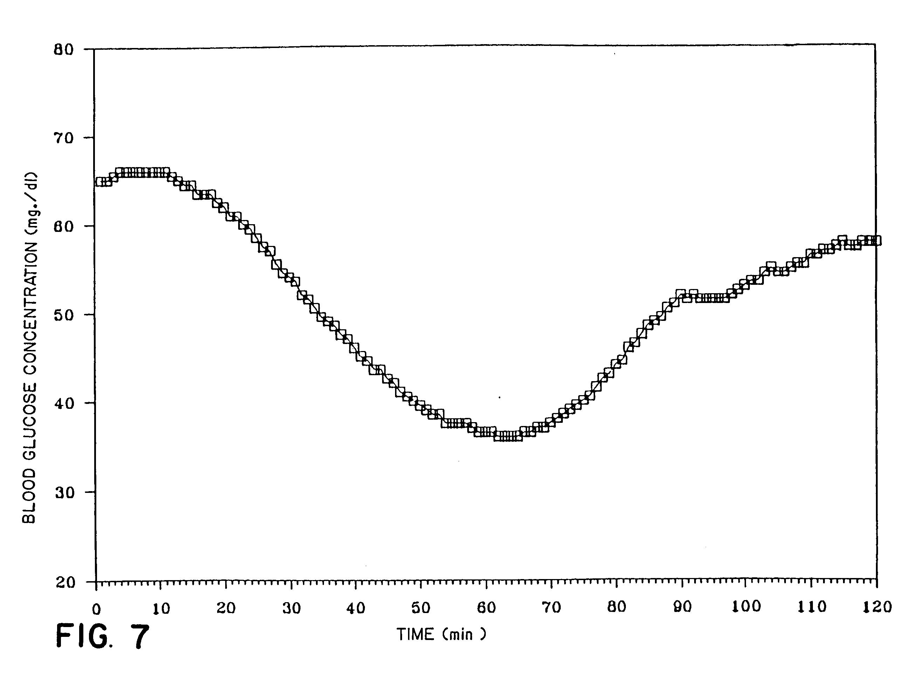

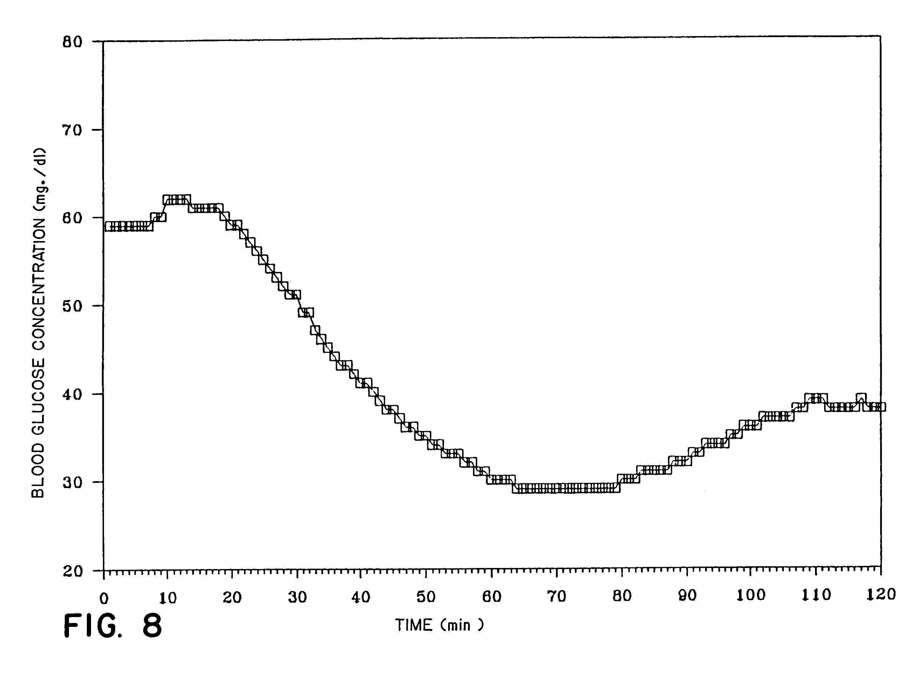

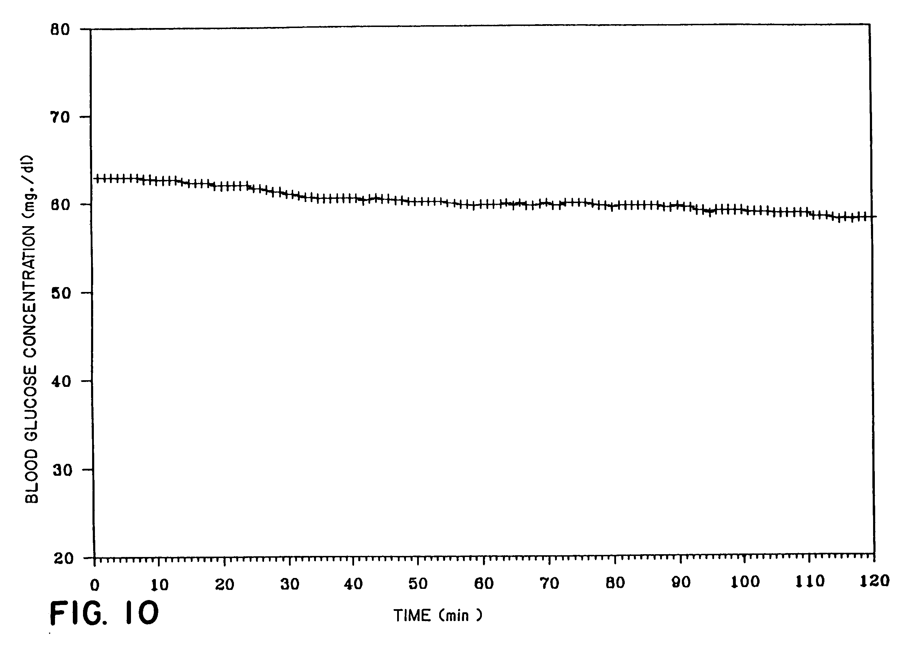

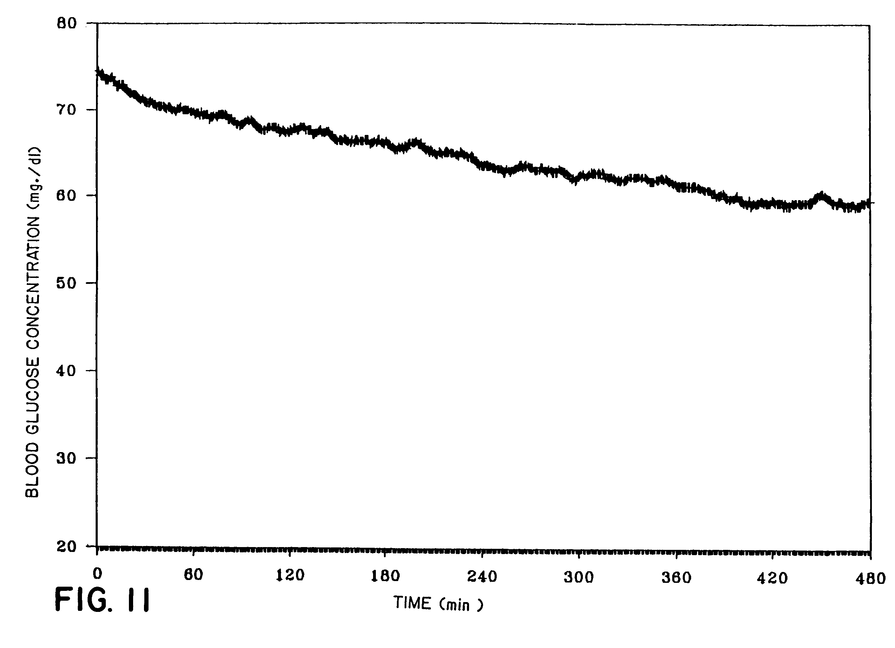

Six preconditioned female mongrel hound dogs treated for worms, weighing 19-20 kg, were fasted overnight and anesthetized in the morning with intraveneous "NEMBUTAL" sodium pentobarbital (250 mg initial dose, 25 mg every 30 minutes for maintenance). Continuous glucose monitoring was then initiated, using the device described above, upon inserting the catheter into one of the major veins of the animal's front leg. A medicine dropper was inserted through the nasal opening into the nasal cavity. A plastic tubing was inserted through the medicine dropper into the nasal cavity. A prepration according to one of the above Examples was administered by injection into the plastic tubing with a small syringe. The solution was then blown into the nasal cavity of the animal. Blood glucose level was continuously monitored. The resulting decrease in blood glucose level for the insulin dosages in Table 1 are recorded in Figures 1 to 12.

## TABLE 1

| Example | Amount of Preparation (ml) | Insulin Dose (Units) | Figure |
|---------|----------------------------|----------------------|--------|
| 1 | 0.1 | 5 | 1 |
| 2 | 0.1 | 5 | 2 |
| 3a | 0.1 | 5 | 3 |
| 3a | 0.2 | 10 | 4 |
| 3b | 0.2 | 10 | 5 |
| 3c | 0.2 | 10 | 6 |
| 8 | 0.2 | 10 | 7 |
| 10 | 0.2 | 10 | 8 |
| 11 | 0.1 | 5 | 9 |
| 11 | 0.2 | 10 | 10 |
| 12 | 0.1 | 5 | 11 |
| 13 | 0.2 | -- | 12 |

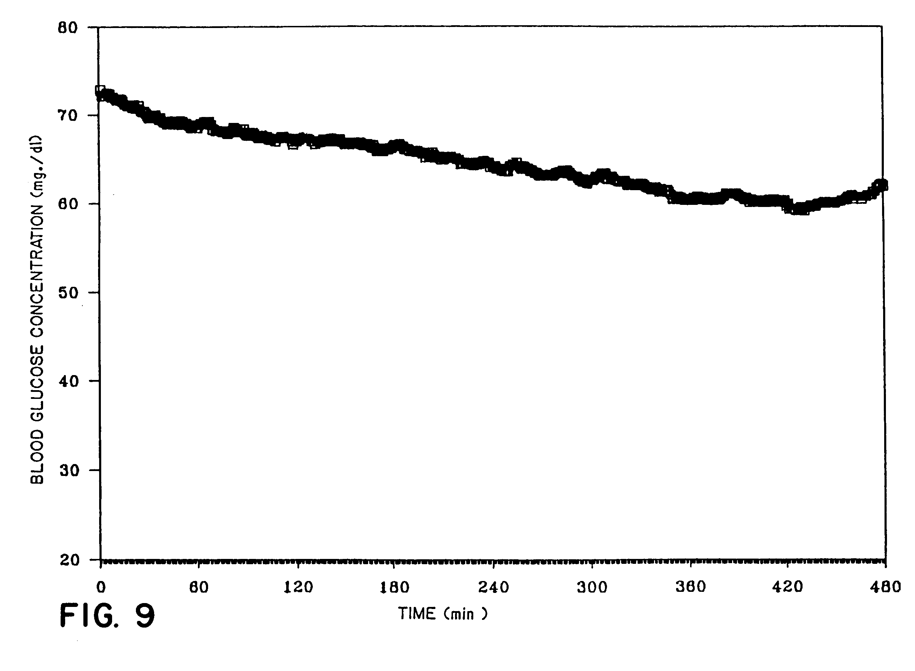

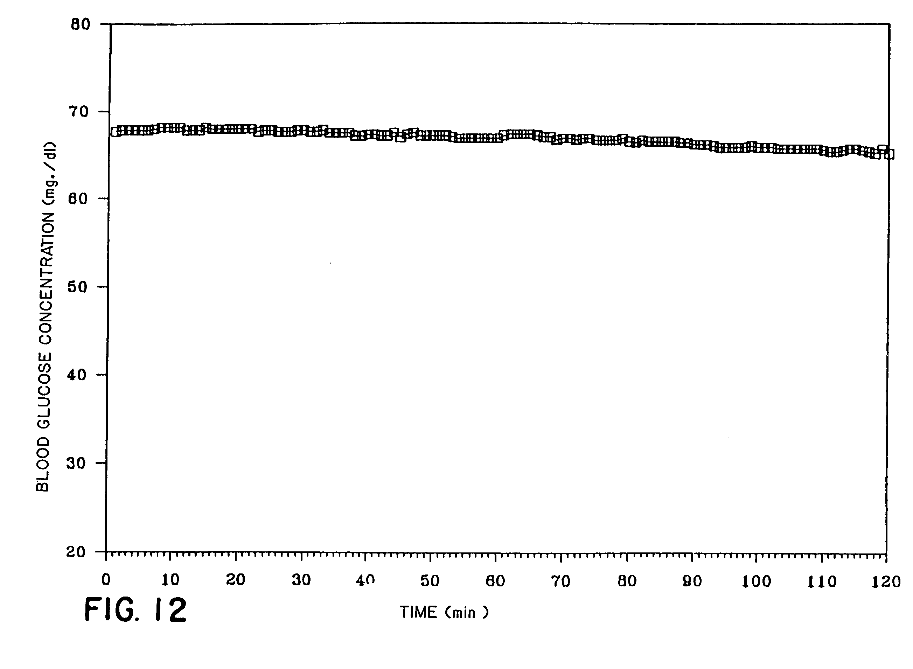

The effectiveness of the insulin preparation of the invention is indicated by a substantial decrease in blood glucose level (Figures 1 – 8) in relation to the comparative examples lacking either glycyrrhetinic acid (Figures 9 – 11) or insulin (Figure 12). The results indicate that glycyrrhetinic acid is effective in enhancing absorption of insulin across the nasal membrane.

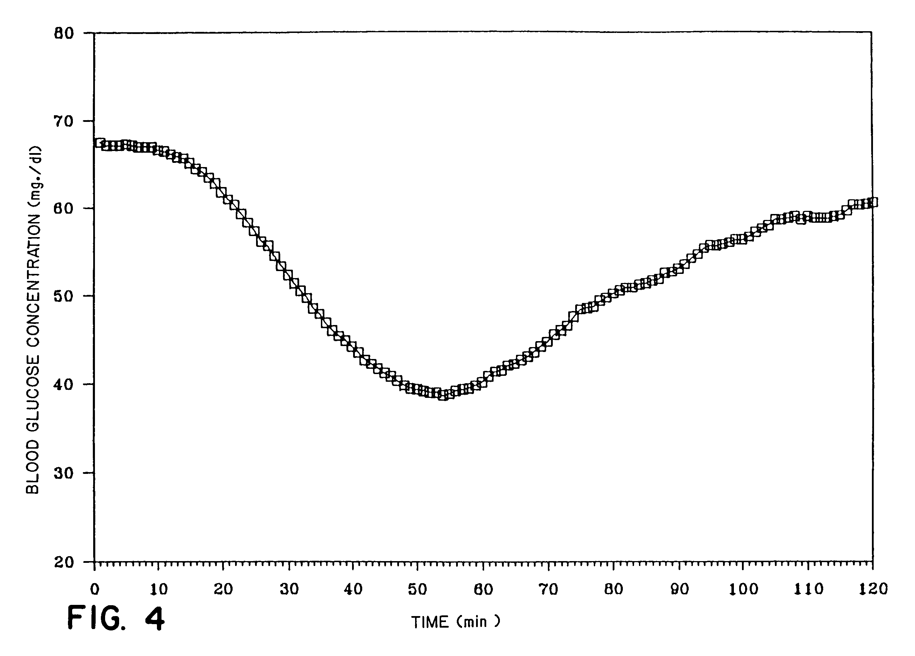

The preparations of the comparative examples lacking glycyrrhetinic acid caused an insignificant decrease in blood glucose from 70 mg% to 60 mg% over seven hours. Preparations containing glycyr – rhetinic acid and five units of insulin induced marked decreases in blood glucose concentration in only 40 to 50 minutes (Figures 1, 2 and 3). The effect was even greater in those animals receiving 10 unit insulin doses (Figures 4, 5, 6, 7 and 8).

Human Study

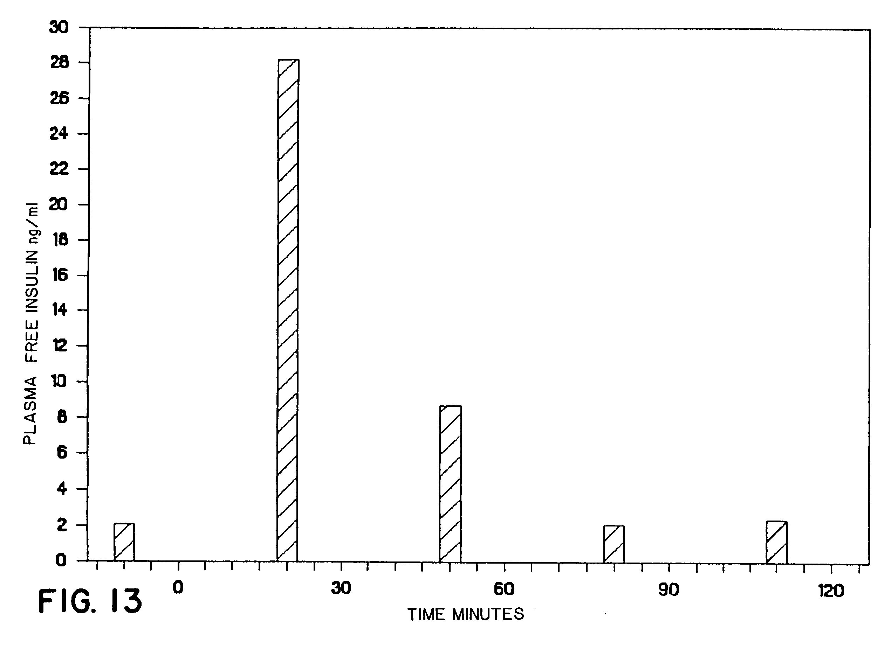

A preparation according to Example 6, except for the substitution of a porcine insulin solution of 50 units/ml insulin (Eli Lilly & Co.) for human insulin, was administered to a diabetic patient as follows. The basal free plasma insulin concentration was measured as 2 ng/ml. The patient received a dose of the preparation equivalent to 50 units of insulin at time T = 0 from a nasal nebulizer (Pfiffer Inc., Princeton Jct, NJ). The tip of the nebulizer was introduced through the nasal aperature. The patient was instructed to inhale as the plunger was depressed, delivering the preparation as a spray of droplets into the upper and inner areas of the nasal cavity. Twenty minutes after administration, the concentration of free insulin in the patient's blood increased to 28 ng/ml. The free insulin level fell to 9 ng/ml 50 minutes after administration, and to the basal level of 2 ng/ml at 80 minutes. See Figure 13. The patient reported no iritation or stinging.

Example 14 – Human Growth Hormone

Example 3a was repeated except that 7 mg of human growth hormone was substituted for insulin.

The present invention nay be embodied in other specific forms without departing from the spirit or essential attributes thereof and, accordingly, reference should be made to the appended claims, rather than to the foregoing specification, as indicating the scope of the invention.

**Claims**

**Claims for the following Contracting States : AT, BE, CH, DE, FR, GB, IT, LU, NL, SE**

1. A composition for the intranasal administration of a pharmaceutically − active substance, characterised in that the composition comprises an effective amount of the pharmaceutically − active substance, a basic salt of an amino acid and glycyrrhetinic acid.

2. A composition according to claim 1, in which the glycyrrhetinic acid is 18 − alpha − glycyrrhetinic acid or 18 − beta − glycyrrhetinic acid.

3. A composition according to claim 1 or 2, in which an amount in the range from 0.25% to 1.0% (w/v) of glycyrrhetinic acid is present.

4. A composition according to claim 1, 2 or 3, in which the molar concentration of glycyrrhetinic acid is equal to the molar concentration of the amino acid basic salt.

5. A composition according to any preceding claim, in which the amino acid basic salt is selected from the sodium and potassium salts of glycine, aspartic acid and glutamic acid.

6. A composition according to any preceding claim, in which a polyhydric alcohol is present.

7. A composition according to claim 6, in which the polyhydric alcohol is glycerin or propylene glycol.

8. A composition according to claim 6 or 7, in which the polyhydric alcohol is present in an amount effective to increase the solubility of the glycyrrhetinic acid in the composition.

9. A composition according to any preceding claim, in which the pharmaceutically − active substance is insulin.

10. A composition according to claim 9, which comprises an aqueous solution.

11. A composition according to any of claims 1 to 8, in which the pharmaceutically − active substance is growth hormone.

12. A composition according to any of claims 1 to 8, in which the pharmaceutically − active substance is glucagon.

13. A composition according to any of claims 1 to 8, in which the pharmaceutically − active substance is adrenocorticotropic hormone.

14. A composition according to any of claims 1 to 8, in which the pharmaceutically − active substance is the delivery of vitamin $B_{12}$.

15. A method of manufacture of a composition for the intranasal administration of a pharmaceutically − active substance, characterised in that the pharmaceutically − active substance, a basic salt of an amino acid and glycyrrhetinic acid are admixed and formed into a solution.

16. A method according to claim 15, in which the ingredients are admixed in any order subject to the formation of an aqueous solution which comprises a composition as defined in any of claims 2 to 14.

**Claims for the following Contracting States : ES, GR**

1. A method of manufacture of a composition for the intranasal administration of a pharmaceutically − active substance, characterised in that the pharmaceutically − active substance, a basic salt of an amino acid and glycyrrhetinic acid are admixed and formed into the composition.

2. A method according to claim 1, in which the glycyrrhetinic acid is 18 − alpha − glycyrrhetinic acid or 18 − beta − glycyrrhetinic acid.

EP 0 285 367 B1

3. A method according to claim 1 or 2, in which the composition is formed to contain an amount in the range from 0.25% to 1.0% (w/v) of glycyrrhetinic acid.

4. A method according to claim 1, 2 or 3, in which the composition is formed to have a molar concentration of glycyrrhetinic acid equal to the molar concentration of the amino acid basic salt.

5. A method according to any preceding claim, in which the amino acid basic salt is selected from the sodium and potassium salts of glycine, aspartic acid and glutamic acid.

6. A method according to any preceding claim, in which the composition is formed also to contain a polyhydric alcohol.

7. A method according to claim 6, in which the polyhydric alcohol is glycerin or propylene glycol.

8. A method according to claim 6 or 7, in which the polyhydric alcohol is present in an amount effective to increase the solubility of the glycyrrhetinic acid in the composition.

9. A method according to any preceding claim, in which the pharmaceutically−active substance is insulin.

10. A method according to claim 9, in which the composition is formed as an aqueous solution.

11. A method according to any of claims 1 to 8, in which the pharmaceutically−active substance is growth hormone.

12. A method according to any of claims 1 to 8, in which the pharmaceutically−active substance is glucagon.

13. A method according to any of claims 1 to 8, in which the pharmaceutically−active substance is adrenocorticotropic hormone.

14. A method according to any of claims 1 to 8, in which the pharmaceutically−active substance is the delivery of vitamin $B_{12}$.

**Patentansprüche**

**Patentansprüche für folgende Vertragsstaaten : AT, BE, CH, DE, FR, GB, IT, LU, NL, SE**

1. Zusammensetzung zur intranasalen Verabreichung einer pharmazeutisch aktiven Substanz, dadurch gekennzeichnet, daß die Zusammensetzung eine wirksame Menge der pharmazeutisch aktiven Substanz, ein Basensalz einer Aminosäure und Glycyrrhetinsäure umfaßt.

2. Zusammensetzung gemäß Anspruch 1, in welcher die Glycyrrhetinsäure 18−alpha−Glycyrrhetinsäure oder 18−beta−Glycyrrhetinsäure ist.

3. Zusammensetzung gemäß Anspruch 1 oder 2, in welcher eine Menge Glycyrrhetinsäure im Bereich von 0,25% bis 1,0% (Gew./Vol.) vorhanden ist.

4. Zusammensetzung gemäß Anspruch 1, 2 oder 3, in welcher die molare Glycyrrhetinsäurekonzentration gleich der molaren Konzentration des Aminosäure−Basensalzes ist.

5. Zusammensetzung gemäß irgendeinem voranstehenden Anspruch, in welcher das Aminosäure−Basensalz aus den Natrium− und Kaliumsalzen von Glycin, Asparaginsäure und Glutaminsäure ausgewählt ist.

6. Zusammensetzung gemäß irgendeinem voranstehenden Anspruch, in welcher ein mehrwertiger Alkohol vorhanden ist.

7. Zusammensetzung gemäß Anspruch 6, in welcher der mehrwertige Alkohol Glycerin oder Propyleng− lykol ist.

9

**8.** Zusammensetzung gemäß Anspruch 6 oder 7, in welcher der mehrwertige Alkohol in einer zum Erhöhen der Löslichkeit der Glycyrrhetinsäure in der Zusammensetzung wirksamen Menge vorhanden ist.

**9.** Zusammensetzung gemäß irgendeinem voranstehenden Anspruch, in welcher die pharmazeutisch aktive Substanz Insulin ist.

**10.** Zusammensetzung gemäß Anspruch 9, welche eine wäßrige Lösung umfaßt.

**11.** Zusammensetzung gemäß einem der Ansprüche 1 bis 8, in welcher die pharmazeutisch aktive Substanz ein Wachstumshormon ist.

**12.** Zusammensetzung gemäß einem der Ansprüche 1 bis 8, in welcher die pharmazeutisch aktive Substanz Glucagon ist.

**13.** Zusammensetzung gemäß einem der Ansprüche 1 bis 8, in welcher die pharmazeutisch aktive Substanz ein adrenocorticotropes Hormon ist.

**14.** Zusammensetzung gemäß einem der Ansprüche 1 bis 8, in welcher die pharmazeutisch aktive Substanz die Zufuhr von Vitamin $B_{12}$ ist.

**15.** Verfahren zur Herstellung einer Zusammensetzung zur intranasalen Verabreichung einer pharmazeu‒ tisch wirksamen Substanz, dadurch gekennzeichnet, daß die pharmazeutisch aktive Substanz, ein Basensalz einer Aminosäure und Glycyrrhetinsäure vermischt und zu einer Lösung verarbeitet werden.

**16.** Verfahren gemäß Anspruch 15, bei welchem die Bestandteile in beliebiger Reihenfolge unter Voraus‒ setzung der Bildung einer wäßrigen Lösung vermischt werden, welche eine wie in einem der Ansprü‒ che 2 bis 14 definierte Zusammensetzung umfaßt.

**Patentansprüche für folgende Vertragsstaaten : ES, GR**

**1.** Verfahren zur Herstellung einer Zusammensetzung zur intranasalen Verabreichung einer pharmazeu‒ tisch aktiven Substanz, dadurch gekennzeichnet, daß die pharmazeutisch aktive Substanz, ein Basen‒ salz einer Aminosäure und Glycyrrhetinsäure vermischt und zu der Zusammensetzung verarbeitet werden.

**2.** Verfahren gemäß Anspruch 1, bei welchem die Glycyrrhetinsäure 18‒alpha‒Glycyrrhetinsäure oder 18‒beta‒Glycyrrhetinsäure ist.

**3.** Verfahren gemäß Anspruch 1 oder 2, bei welchem die Zusammensetzung so beschaffen ist, daß sie eine Menge Glycyrrhetinsäure im Bereich von 0,25% bis 1,0% (Gew./Vol.) enthält.

**4.** Verfahren gemäß Anspruch 1, 2 oder 3, bei welchem die Zusammensetzung so beschaffen ist, daß sie eine molare Glycyrrhetinsäurekonzentration enthält, welche gleich der molaren Konzentration des Aminosäure‒Basensalzes ist.

**5.** Verfahren gemäß irgendeinem voranstehenden Anspruch, bei welchem das Aminosäure‒Basensalz aus den Natrium‒ und Kaliumsalzen von Glycin, Asparaginsäure und Glutaminsäure ausgewählt ist.

**6.** Verfahren gemäß irgendeinem voranstehenden Anspruch, bei welchem die Zusammensetzung so beschaffen ist, daß sie auch einen mehrwertigen Alkohol enthält.

**7.** Verfahren gemäß Anspruch 6, bei welchem der mehrwertige Alkohol Glycerin oder Propylenglykol ist.

**8.** Verfahren gemäß Anspruch 6 oder 7, bei welchem der mehrwertige Alkohol in einer zum Erhöhen der Löslichkeit der Glycyrrhetinsäure wirksamen Menge vorhanden ist.

**9.** Verfahren gemäß irgendeinem voranstehenden Anspruch, bei welchem die pharmazeutisch aktive Substanz Insulin ist.

**10.** Verfahren gemäß Anspruch 9, bei welchem die Zusammensetzung als wäßrige Lösung ausgebildet ist.

**11.** Verfahren gemäß einem der Ansprüche 1 bis 8, bei welchem die pharmazeutisch aktive Substanz ein Wachstumshormon ist.

**12.** Verfahren gemäß einem der Ansprüche 1 bis 8, bei welchem die pharmazeutisch aktive Substanz Glucagon ist.

**13.** Verfahren gemäß einem der Ansprüche 1 bis 8, bei welchem die pharmazeutisch aktive Substanz ein adrenocorticotropes Hormon ist.

**14.** Verfahren gemäß einem der Ansprüche 1 bis 8, bei welchem die pharmazeutisch aktive Substanz die Zufuhr von Vitamin $B_{12}$ ist.

**Revendications**

**Revendications pour les Etats contractants suivants : AT, BE, CH, DE, FR, GB, IT, LU, NL, SE**

**1.** Composition pour l'administration intranasale d'une substance pharmaceutiquement active, composition caractérisée en ce qu elle comprend une quantité utile de la substance pharmaceutiquement active, un sel basique d'un acide aminé et de l'acide glycyrrhétinique.

**2.** Composition selon la revendication 1, caractérisée en ce que l'acide glycyrrhétinique est de l'acide 18 − alpha − glycyrrhétinique ou de l'acide 18 − bêta − glycyrrhétinique.

**3.** Composition selon l'une quelconque des revendications 1 ou 2, caractérisée en ce qu'on utilise une quantité d'acide glycyrrhétinique se situant dans la plage de 0,25 % à 1,0 % (poids/volume).

**4.** Composition selon l'une quelconque des revendications 1 à 3, caractérisée en ce que la concentration molaire de l'acide glycyrrhétinique est égale à la concentration molaire du sel basique de l'acide aminé.

**5.** Composition selon l'une quelconque des revendications précédentes, caractérisée en ce que le sel basique de l'acide aminé est choisi parmi les sels de sodium et de potassium de la glycine, de l'acide aspartique et de l'acide glutamique.

**6.** Composition selon l'une quelconque des revendication précédentes, caractérisée en ce qu'on utilise de l'alcool polyhydrique.

**7.** Composition selon la revendication 6, caractérisée en ce que l'alcool polyhydrique est de la glycérine ou du glycol de propylène.

**8.** Composition selon l'une quelconque des revendications 6 ou 7, caractérisée en ce que l'alcool polyhydrique est présent dans une proportion convenable pour augmenter la solubilité de l'acide glycyrréthinique dans la composition.

**9.** Composition selon l'une quelconque des revendications précédentes, caractérisée en ce que la substance pharmaceutiquement active est de l'insuline.

**10.** Composition selon la revendication 9, caractérisée en ce qu'elle comprend une solution aqueuse.

**11.** Composition selon l'une quelconque des revendications 1 à 8, caractérisée en ce que la substance pharmaceutiquement active est de l'hormone de croissance.

**12.** Composition selon l'une quelconque des revendications 1 à 8, caractérisée en ce que la substance pharmaceutiquement active est du glucagon.

**13.** Composition selon l'une quelconque des revendications 1 à 8, caractérisée en ce que la substance pharmaceutiquement active est de l'hormone adrénocorticotropique.

**14.** Composition selon l'une quelconque des revendications 1 à 8, caractérisée en ce que la substance pharmaceutiquement active est la source de vitamine $B_{12}$.

**15.** Procédé de fabrication d'une composition pour l'administration intra – nasale d'une substance pharma – ceutiquement active, procédé caractérisé en ce que la substance pharmaceutiquement active, un sel basique d'un acide aminé et l'acide glycyrrhétinique, sont ajoutés en mélange et formés en solution.

**16.** Procédé selon la revendication 15, caractérisé en ce que les ingrédients sont ajoutés en mélange dans n'importe quel ordre sous réserve de la formation d'une solution aqueuse comprenant une composition telle que celle définie dans l'une quelconque des revendications 2 à 14.

**Revendications pour les Etats contractants suivants : ES, GR**

**1.** Procédé de fabrication d'une composition pour l'administration intra – nasale d'une substance pharma – ceutiquement active, procédé caractérisé en ce que la substance pharmaceutiquement active, un sel basique d'un acide aminé et de l'acide glycyrrhétinique, sont ajoutés en mélange pour former la composition.

**2.** Procédé selon la revendication 1, caractérisé en ce que l'acide glycyrrhétinique est de l'acide 18 – alpha – glycyrrhétinique ou de l'acide 18 – bêta – glycyrrhétinique.

**3.** Procédé selon l'une quelconque des revendications 1 et 2, caractérisé en ce que la composition est formée de manière à contenir une quantité d'acide glycyrrhétinique se situant dans la plage de 0,25 % à 1,0 % (poids/volume).

**4.** Procédé selon l'une quelconque des revendications 1 à 3, caractérisé en ce que la composition est formée de manière à présenter une concentration molaire d'acide glycyrrhétinique égale à 1 concen – tration molaire du sel basique de l'acide aminé.

**5.** Procédé selon l'une quelconque des revendications précédentes, caractérisé en ce que le sel basique de l'acide aminé est choisi parmi les sels de sodium et de potassium de la glycine, de l'acide aspartique et de l'acide glutamique.

**6.** Procédé selon l'une quelconque des revendications précédentes, caractérisé en ce que la composition est également formée de manière à contenir un alcool polyhydrique.

**7.** Procédé selon la revendication 6, caractérisé en ce que l'alcool polyhydrique est de la glycérine ou du glycol de propylène.

**8.** Procédé selon l'une quelconque des revendications 6 ou 7, caractérisé en ce que l'alcool polyhydrique est présent dans une proportion convenable pour augmenter la solubilité de l'aide glycyrrhétinique dans la composition.

**9.** Procédé selon l'une quelconque des revendications précédentes, caractérisé en ce que la substance pharmaceutiquement active est de l'insuline.

**10.** Procédé selon la revendication 9, caractérisé en ce que la composition est réalisée sous la forme d'une solution aqueuse.

**11.** Procédé selon l'une quelconque des revendications 1 à 8, caractérisé en ce que la substance pharmaceutiquement active est de l'hormone de croissance.

**12.** Procédé selon l'une quelconque des revendications 1 à 8, caractérisé en ce que la substance pharmaceutiquement active est du glucagon.

**13.** Procédé selon l'une quelconque des revendications 1 à 8, caractérisé en ce que la substance pharmaceutiquement active est de l'hormone adrénocorticotropique.

**14.** Procédé selon l'une quelconque des revendications 1 à 8, caractérisé en ce que la substance pharmaceutiquement active est la source de vitamine $B_{12}$.

FIG. I

EP 0 285 367 B1

FIG. 2

EP 0 285 367 B1

FIG. 3

EP 0 285 367 B1

FIG. 4

EP 0 285 367 B1

FIG. 5

FIG. 6

TIME (min.)

BLOOD GLUCOSE CONCENTRATION (mg./dl)

EP 0 285 367 B1

FIG. 7

EP 0 285 367 B1

FIG. 8

FIG. 9

EP 0 285 367 B1

FIG. 10

BLOOD GLUCOSE CONCENTRATION (mg./dl)

TIME (min )

FIG. II

EP 0 285 367 B1

FIG. 12

FIG. 13

EP 0 285 367 B1